# EUROPEAN PATENT APPLICATION

(11) **EP 4 574 048 A1**
(43) Date of publication of application: **25.06.2025**
(21) Application number: 24154900.5
(22) Date of filing: 31.01.2024
(51) Int. Cl.: A61B 6/03, A61B 6/00, G06T 11/00, A61B 5/08

(54) **MEDICAL IMAGE DATA ACQUISITION WITH RADIATION SOURCE CONTROL**

(30) Priority: 19.12.2023 US 202363611794 P
(71) Applicant: Koninklijke Philips N.V., 5656 AG Eindhoven (NL)
(72) Inventor: KABUS, Sven, Eindhoven (NL); PERKINS, Amy, Eindhoven (NL); KRUIS, Matthijs Ferdinand, 5656AG Eindhoven (NL)
(74) Representative: Philips Intellectual Property & Standards

(57) **Abstract**

A method for acquiring medical image data of a subject and a system for controlling an imaging device to acquire medical image data of a subject are provided, as well as an imaging arrangement and a computer program product to carry out the method.

The method comprises receiving a respiratory signal of the subject and correlating the respiratory signal to a position of a support for the subject to identify one or more breathing index coverage matrix elements, and performing an imaging scan of the subject. During the imaging scan the subject is exposed to radiation emitted by a radiation source, which radiation source is controlled by control instructions. The method also comprises determining fill factors of the identified one or more breathing index coverage matrix elements; and updating the radiation source control instructions using the determined fill factors.

## Description

### FIELD OF THE INVENTION

The invention generally relates to medical image data acquisition. In particular, but not exclusively, the invention relates to computed tomography image data acquisition.

### BACKGROUND OF THE INVENTION

In medical imaging, techniques such as computed tomography (CT) and magnetic resonance imaging (MRI) are used to visualize patient anatomy. A CT imaging system generally includes a source of radiation such as x-ray radiation mounted on a rotatable gantry opposite a detector array including one or more rows of detector pixels. The x-ray source rotates around an examination region located between the x-ray tube and the detector array and emits radiation that traverses the examination region and a subject disposed therein. The detector array detects radiation that traverses the examination region and generates projection data indicative of the examination region and the object or subject disposed therein. The projection data is reconstructed to generate volumetric image data indicative of the subject. The volumetric image data can be processed to generate one or more images that include the scanned portion of the subject.

Respiratory gated CT imaging, also referred to as 4D-CT imaging, is routinely used in radiation oncology. In 4D-CT imaging, a series of 3D volumetric images of the subject are acquired over a period of time. This type of imaging can support visualization of the motion of patient anatomy. This is particularly helpful when a region of interest, such as a tumor, is subject to regular and involuntary motion such as respiratory and cardiac motion. On the basis of a respiratory phase based gating, the motion of a lung tumor can be followed over respiratory time.

For 4D-CT imaging of sufficient diagnostic quality, data acquisition takes several minutes. The dose received by the patient during the scan is relatively high. In addition, patients can have irregular breathing and disruptive incidents such as coughing. This can cause under estimation of motion of a tumor and can lead to compromised image quality. Because compromised image quality will lead to a higher radiation dose to the patient during radiation therapy treatment, radiologists in practice opt to immediately acquire a second full diagnostic scan to ensure quality. In severe cases, a full re-scan can be required.

In the publication "Objective function to obtain multiple representative waveforms for a novel helical CT scan protocol" by D. Ruan et al., Med Phys 42(3), 2015, 1164-69, the authors propose the use of objective functions for selecting multiple representative respiratory waveforms. The selection is used to reduce the number of swiping scans in a novel fast helical CT scan protocol to achieve dose reduction.

However, this approach requires a long processing runtime for the proposed model and assumes a regular breathing pattern. While some dose reduction may be achieved, it still cannot account for irregular breathing and abrupt changes in the patient's respiration.

### SUMMARY OF THE INVENTION

The current invention seeks to provide an approach for medical image acquisition with improved image quality for subjects with irregular breathing. The current invention additionally or alternatively seeks to provide an approach for medial image acquisition with reduced radiation exposure for the subject.

Thereto a method for acquiring medical image data of a subject and a system for controlling an imaging device to acquire medical image data of a subject are provided, as well as an imaging arrangement for acquiring medical image data of a subject and a computer program product comprising instructions for causing a processor to carry out the method for acquiring medical image data.

The method for acquiring medical image data comprising steps of receiving a respiratory signal of the subject and correlating the respiratory signal to a position of a support for the subject to identify one or more breathing index coverage matrix elements (BICM); and performing an imaging scan of the subject, during which imaging scan the subject is exposed to radiation emitted by a radiation source, which radiation source is controlled by control instructions. The method also comprises determining fill factors of the identified one or more breathing index coverage matrix elements; and updating the radiation source control instructions using the determined fill factors. The method is preferably computer-implemented or implemented by other suitable calculation means.

In an embodiment of the method determining the fill factors of the one or more breathing index coverage matrix elements and updating the radiation source control instructions using the determined fill factor take place at least partly simultaneously with performing the imaging scan of the subject.

Preferably, the method steps are repeated until the breathing index coverage matrix meets a stopping criterium. According to a further option, the stopping criterium is met when each fill factor has reached a coverage threshold.

According to one aspect, the radiation source control instructions are instructions for switching the source on or off. In this way, the subject is only exposed to radiation when data needs to be acquired. Alternatively to switching off the radiation source, the radiation source control instructions can be instructions to block the x-ray beam. This can, for example, be done by inserting a shutter into the beam. In a further alternative, the radiation source control instructions can be instructions for attenuating the x-ray beam. Attenuating the x-ray beam allows for a more gradual change in radiation intensity.

According to another aspect, the method further comprises a step of updating the imaging scan pitch and/or scan lengths using the determined fill factors. When additional data acquisition is only required for a limited number of positions of the subject support, the scan speed and length can be adjusted to perform a scan that covers those positions. This provides for more efficient image data acquisition.

In one option, the fill factor has a number of discrete values. Using a number of discrete values is computationally efficient.

In another option, the fill factor comprises dwell time. This example can be particularly advantageously combined with simultaneously determining the fill factors of the BICM elements and updating the radiation source control instructions using the determined fill factors. Certain breathing states such as the maximum exhale state are likely to last for a longer period of time than others. The amount of time spent in the corresponding BICM elements can therefore be longer than needed for sufficient data acquisition. By using dwell time as a fill factor and updating radiation source control instructions to switch off or reduce the amount of radiation once sufficient time has been spent to acquire data, additional dose exposure for the subject can be reduced.

In a further embodiment of the method, wherein the radiation source is controlled to stop emitting radiation when irregular breathing is detected and to resume emitting radiation when regular breathing resumes. When the subject breathes irregularly, for example during coughing, any data that is gathered cannot be used to reconstruct a medical image of sufficient quality. The current method enables updating of the control instructions to switch off the radiation source and reduce the additional dose to the patient. Moreover, fill factors of the BICM elements can be used to limit any further scans to acquire only the additional imaging data needed instead of acquiring a full additional scan of the subject.

The arrangement for acquiring a computed tomography image of a subject comprises an imaging device comprising a controllable source of radiation and the above-described system for controlling the imaging device. Preferably, the arrangement further comprises a respiratory monitor configured to provide the respiratory signal of the subject. It is also an option that the arrangement comprises an image reconstruction system configured to reconstruct the imaging data to generate an image of the subject disposed in the examination region. The generated image may be viewed and used by a physician for diagnosis and/or treatment of the subject.

The computer program product comprises instructions that cause a processor to carry out the above described method, when the computer program is executed.

An advantage of the current invention is that image quality, and in particular the quality of a 4D-CT image, can be improved for subjects with irregular breathing. By determining the fill factors, combinations of position and respiratory state where insufficient data has been acquired are known during data acquisition and additional data can be acquired as part of the image acquisition process. An additional advantage is that this could be done without the need to reconstruct the medical image first, thereby also reducing the need to recall patients for additional medical scans.

Another advantage is that the radiation dose to which the subject is exposed can be reduced. By determining the fill factors, the invention monitors the data acquisition for a combination of position and respiratory state. When sufficient data has been collected, the radiation source can be switched off for this. This avoids unnecessary further exposure without compromising image quality. Alternatively or additionally, the radiation source can be switched off when irregular breathing or coughing is detected and data acquisition can resume when the subject resumes regular breathing. Data acquired during an episode of irregular breathing can compromise the quality of image and therefore would need to be reacquired. By switching off the radiation source, additional exposure of radiation is reduced.

### BRIEF DESCRIPTION OF THE FIGURES

In the following drawings:
Fig. 1 schematically and exemplarity illustrates an imaging arrangement for acquiring CT image data of a subject comprising a system for controlling a CT imaging device.
Fig. 2 schematically illustrates an example of a method for acquiring medical image data of a subject.
Figs 3a and 3b schematically illustrate examples of a breathing index coverage matrix.
Figs 4a and 4b schematically illustrate examples of a breathing index coverage matrix for a low-pitch helical CT scan.
Figs 5a and 5b schematically illustrate examples of a breathing index coverage matrix for repeated scans in a high-pitch helical CT scan.

### DETAILED DESCRIPTION OF THE INVENTION

In the examples below, medical image data is acquired for a subject. Such a subject can be a human being, particularly a human patient requiring imaging for medical purposes. However, alternative subjects are also envisaged, for example animals such as research animals, pets, or livestock. The approach according to the invention can also be applied to inanimate objects having intrinsic periodic motion that cannot be paused during image acquisition. In the examples, the imaging device is illustrated as a computed tomography (CT) scanner, but other devices may also be used to acquire the medical image data.

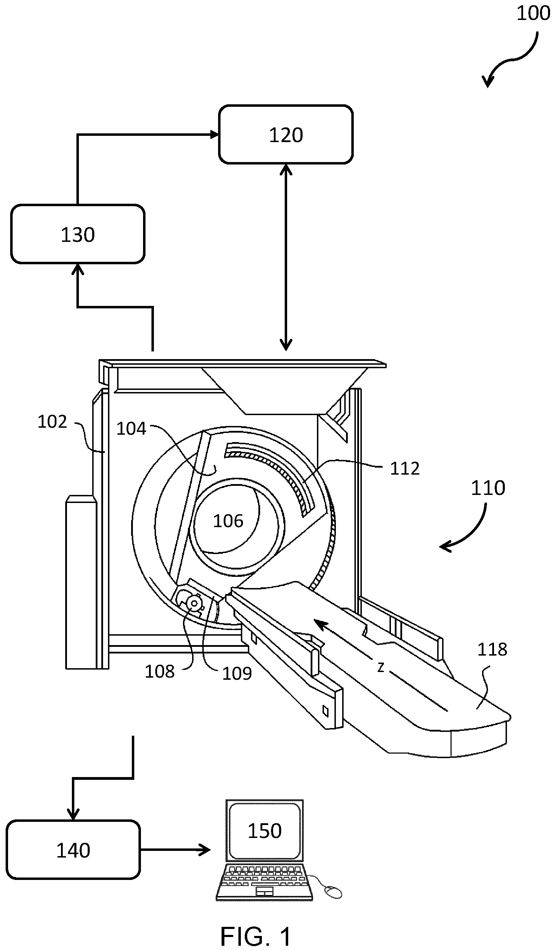

Fig. 1 illustrates an imaging arrangement 100 for acquiring medical image data of a subject.

The imaging arrangement 100 comprises an imaging apparatus 110. The imaging apparatus in this exemplary embodiment is a CT scanner. The computed tomography imaging apparatus 110 includes a stationary gantry 102 and a rotating gantry 104, which is rotatably supported by the stationary gantry 102. When the system is in operation, the rotating gantry 104 rotates around an examination region 106 about a longitudinal or z-axis. A radiation source 108, such as an x-ray tube, is supported by and rotates with the rotating gantry 104 and emits radiation that traverses the examination region 106. A source collimator 109 collimates the emitted radiation to form a generally fan, wedge, or cone shaped radiation that traverses the examination region 106.

The radiation source 108 is the type where the amount of radiation that is emitted can be controlled during the acquisition of the image data.

A support 118, such as a bed or couch, is provided to support an object or subject to be imaged in the examination region 106. Support 118 is movable along the z-axis in coordination with the rotation of the rotating gantry 104 to facilitate the desired scanning trajectory, preferably a helical scanning trajectory.

A radiation sensitive detector array 112 detects radiation emitted by the radiation source 108 that traverses the examination region 106 and generates projection image data indicative of the detected radiation. The illustrated radiation sensitive detector array 112 includes one or more rows of radiation sensitive photosensor pixels along the z-axis.

In the imaging arrangement, a respiratory motion signal 130 of the subject is provided. The respiratory motion can be the actual breathing motion of a human or animal subject, but can also be an alternative type of regular motion with properties comparable to breathing motion. The respiratory motion signal can, for example, be a measured breathing amplitude or a breathing phase. The imaging arrangement preferably comprises a respiratory monitor to measure the respiratory motion signal 130. The respiratory monitor can be a pressure belt attached to the subject, which measures chest motion. Alternatively, a camera-based method can be used. For example, a depth camera mounted in the ceiling of the examination room or on the imaging device 110 itself can be used to monitor the chest motion of the subject. Camera-based methods have the advantages of being more efficient in workflow for the scan technicians and of being contact-free for the subject.

In this example, the imaging arrangement 100 further comprises an optional image reconstruction system 140, which reconstructs the projection data and generates volumetric image data indicative of the examination region 106, including structure of an object or subject disposed therein. One or more images can be generated from the volumetric image data. The imaging arrangement 100 also comprises an optional display 150, for displaying the one or more generated images.

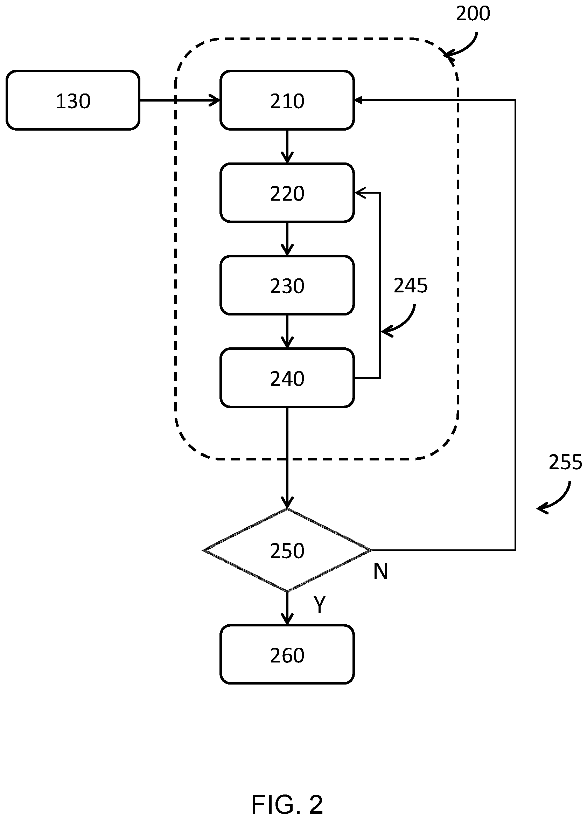

The imaging arrangement 100 further comprises a system 120 for controlling the imaging device 110, in this example the CT imaging device, to acquire the medical image data of the subject. For this purpose, the control system 120 comprises a control unit which is configured to perform a method for acquiring medical image data which will be described in further detail with reference to Fig. 2.

Fig. 2 schematically illustrates an example of a method for acquiring medical image data 200, for example CT image data, of a subject.

The method for acquiring medical image data 200 comprising steps of receiving a respiratory signal 130 of the subject and correlating the respiratory signal to a position of a support for the subject to identify one or more breathing index coverage matrix (BICM) elements 210; and performing an imaging scan of the subject 220, during which imaging scan the subject is exposed to radiation emitted by a radiation source, which radiation source is controlled by control instructions. The method also comprises determining fill factors of the identified one or more BICM elements 230; and updating the radiation source control instructions using the determined fill factors 240.

In an embodiment of the method determining the fill factors of the one or more BICM elements 230 and updating the radiation source control instructions using the determined fill factor 240 take place at least partly simultaneously with performing the imaging scan of the subject 220. These steps can, for example, be performed simultaneously by parallel processing. Fig. 2 shows an alternative option where the steps of determining the fill factors 230, updating the source control instructions and performing the imaging scan 220 take place iteratively 245. While imaging data is acquired from the scan, the fill factor of the current BICM element is determined. This fill factor is then used to update the radiation source control instructions 240 and the scan continues 220 with the updated instructions. Performing these steps simultaneously allows for additional dose saving for the subject, because the source of the x-ray radiation can be switched off during the scan as soon as sufficient data has been gathered for a BICM element.

Another preferred embodiment that can be used separately or in combination with other options is also illustrated in Fig. 2. The method steps are repeated 255 until the BICM meets a stopping criterium 250. When the stopping criterium is met, the method is completed 260. The stopping criterium can be defined for the BICM as a whole. The criterium can, for example, prescribe a predefined portion of the fill factors reaching a coverage threshold. Alternatively, the stopping criterium can be met when each fill factor has reached a coverage threshold. Such a coverage threshold can be the same for each fill factor, but can also be defined individually for each BICM element. The stopping criterium can be pre-defined before acquisition of the medical image data starts. Alternatively, the stopping criterium and/or the coverage thresholds for the fill factors can be adjusted during or after the scan of the subject is performed.

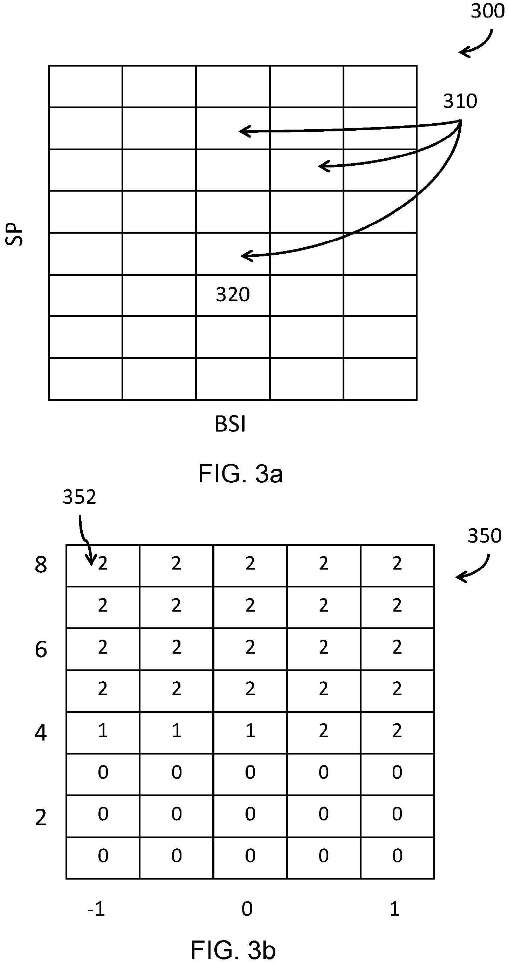

Fig. 3a schematically illustrates an example of a breathing index coverage matrix (BICM) 300. The horizontal axis of the matrix 300 is defined by the breathing state index BSI. The BSI is derived from the respiratory motion signal and can, for example, be the breathing amplitude or breathing phase. The BSI will vary between the maximum inhale and maximum exhale states of the subject. The BSI can, for example, be represented in the matrix in actual measured units, or as normalized units with -1 for the maximum exhale state and +1 for the maximum inhale state. The vertical axis of the matrix 300 is defined by the position of the support 118 for the subject. For a CT scan this position is commonly referred to as the axial position of the subject or the slice number of the acquired CT image.

The BICM has BICM elements 310. Each BICM element represents a specific combination of a position of the subject support with a breathing state. Each BICM element has a fill factor 320 that indicates the image data that has been acquired for the BICM element. For example, fill factor 320 can be the amount of data that has been acquired for the BICM element. Fill factor 320 can also be the dwell time in the BICM element, meaning the amount of time spent acquiring data at this combination of support position and breathing state. Alternatively, the fill factor 320 can have a number of discrete values. In a particularly simple embodiment this can be 0 for no data, or insufficient data acquired and 1 for enough data acquired. In an alternative option, the discrete values can be the number of breaths that have passed through the BICM. The fill factor can also comprise multiple components, such as an inhale and an exhale component. In this way, the fill factor can indicate separately the amount of data that has been acquired in the inhale and exhale motions of the subject.

Fig. 3b schematically illustrates another example of a BICM 350. In this example, the BSI is a normalized respiratory amplitude where -1 corresponds to the maximum exhale amplitude, 0 corresponds to the mid in- or exhale amplitude and 1 corresponds to the maximum inhale amplitude. The matrix of Fig. 3b is divided into five values for the BSI, but this is merely done for illustration purposes. The number of values used for the BSI can be adjusted to the required image quality. Typically, 8-10 respiratory gates are used in 4D-CT image reconstruction, and therefore the BSI will typically have at least 8-10 values, and preferably more than 10. The position of the support for the subject is indicated as slice number. For illustration purposes, this BICM has eight slices. However, this number may be different in practice. A CT scan of the chest including the lungs will typically have 100-200 slices. The BICM elements have fill factors with three different discrete values. The fill factor is 0 when no data has been acquired, the fill factor is 1 when data has been acquired during either the inhale or the exhale phase, and the fill factor is 2 when data has been acquired during both the inhale and the exhale phase.

In Fig. 3b, the fill factors of the BICM elements are determined during the imaging scan. In this example, the scan starts with slice 8, then moves downwards and ends after slice 1. The scan starts with the breathing state of the subject at maximum exhale, which is the top left corner BICM element 352. All BICM elements initially have a fill factor of 0. As the subject inhales and exhales, the fill factors are first updated to 1 and then to 2. In the BICM of Fig. 3b, data has been acquired for both breathing phases for all matrix elements of slices 5-8 and all fill factors for these elements are now 2. Data acquisition for slice 4 is in progress and part of fill factors are determined as 1, while part have been further determined as 2 and updated to this value. No data has been acquired yet for slices 1-3 and the fill factors are still 0.

In this example, sufficient data for a high-quality medical image has been acquired when the fill factor is 2. The radiation source of the CT scanner should be switched off when enough data has been acquired, but remain on otherwise. Therefore, the radiation source control instructions are updated to switch the source off for BICM elements where the fill factor is 2. When each BICM element fill factor is 2, the coverage threshold has been reached and image data acquisition is complete. The image acquisition illustrated in Fig. 3b can be complete after one imaging scan of the subject has been performed. However, it can also occur that not all of the fill factors of the BICM elements reach a value of 2, for example when the subject takes shallow breaths during the acquisition of slices 1 and 2. In that case, a second imaging scan of the subject can be performed. The additional scan can be initialized with the updated radiation source control instruction without the need to first reconstruct the image and check this for quality. This second scan can be performed by moving the subject in the same direction, moving from slice 8 down to 1. More advantageously this will be done in the opposite direction, moving from slice 1 up to 8 since this will require less motion of the subject support. In both cases substantial dose will be saved because the radiation source will only be switched on to emit radiation for those BICM elements where additional data is needed.

In an alternative option to requiring each filling factor to reach a coverage threshold, the stopping criterium for the image data acquisition could be to require a predefined portion, such as 90%, of the fill factors to reach the coverage threshold. In a further alternative, the stopping criterium for the image data acquisition can be to require that the fill factors of certain selected BICM elements meet the coverage criterium.

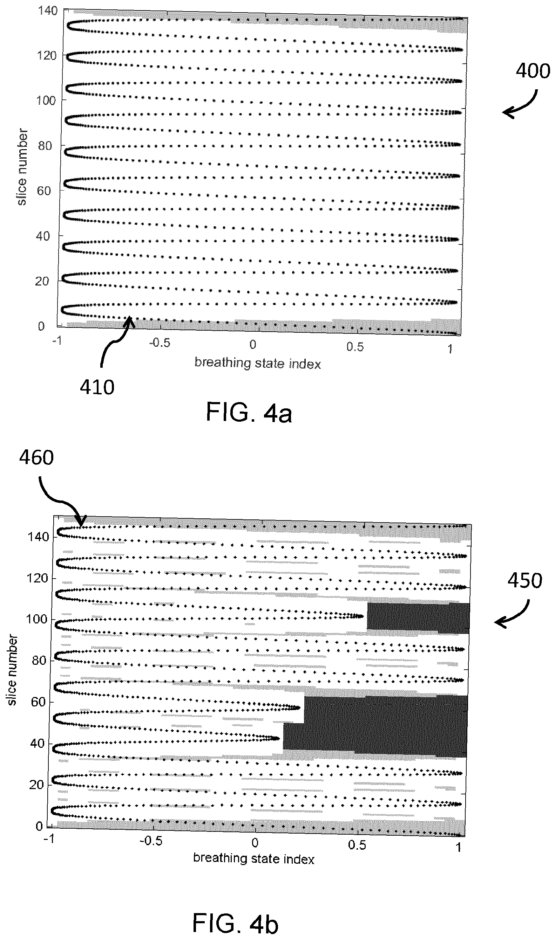

Fig. 4a schematically illustrates an example of a breathing index coverage matrix 400 for a low-pitch helical CT scan. In the BICM 400 of Fig. 4a, the position of the subject support is indicated as slice number with 140 slices. The breathing state index is provided in normalized units with -1 being the maximum exhale state and 1 being the maximum inhale state. The BICM 400 is illustrated with an overlay of the respiratory signal 410. The respiratory signal shown in this Figure is an idealized representation with a fully regular breathing period and equal amplitude in each breath. The respiratory signal 410 is plotted with dots that are equidistant in time.

In the BICM of Fig. 4a, all BICM elements have been identified and their fill factors determined. A fill factor here can have three discrete values: no data acquired, data acquired either during inhalation or exhalation, and data acquired during both inhalation and exhalation. When data has been gathered for both inhalation and exhalation, the BICM is shown as white. When data has been gathered for either inhalation or exhalation, the BICM is shown as grey. This can be seen for part of the first five slices, where the subject only exhales, and part of the last six slices, where the subject only inhales. There are no BICM elements where no data was acquired. In this example, the coverage threshold for the BICM elements for slices 1-10 and 131-140 is "grey", and the coverage threshold for the BICM elements for slices 11-130 is "white". All fill factors have reached their threshold and the BICM therewith meets the stopping criterium. This means no additional data is needed and a single pass of the support with the subject through the scanner is sufficient.

The plotted dots of the respiratory signal in Fig. 4a are equidistant in time. It is immediately visible that the amount of time spent at a certain BICM element is not equal for all elements. This is inherent to breathing motion, where more time is spent at the maximum inhale point, and even more at the maximum exhale point. The time spent at each BICM element is also referred to as the dwell time. The dwell time in BICM elements with breathing stat indices between -0.5 and 0.5 is sufficient for a high quality 4D-CT image. It follows that for the BICM elements with breathing state indices approaching -1 and 1, the source of radiation need not be on continuously to acquire sufficient data. Alternatively, or additionally, the fill factors of the BICM elements can therefore also have a coverage threshold for dwell time. Once the threshold has been reached, the control instructions for the radiation source can be switched to "off'. This allows for substantial reduction of up to 50% of the radiation dose the subject is exposed to.

Fig. 4b schematically illustrates another example of a breathing index coverage matrix 450 for a low-pitch helical CT scan. For this CT scan the same acquisition time was used, but with a larger scan length. The additional slices as can be seen on the vertical axis of the BICM 450 of Fig. 4b. With the extended scan length for the same acquisition time, the pitch of this helical CT scan is slightly higher than that of the scan illustrated in Fig. 4a. This BICM 450 is also illustrated with an overlay of the respiratory signal 460 plotted with dots equidistant in time. In this example the subject shows irregular breathing. The breathing period is regular in time, but the subject took some shallow breath inhales for breathing cycles 3, 4 and 7, visible in slices 39-68 and 99-113.

The fill factors of the BICM elements are illustrated in the same manner as for Fig. 4a. The BICM elements where no data has been acquired are shown as black. Because the pitch of this scan is slightly higher, some more gray areas can now be seen where data has only been acquired during either the inhale or the exhale phase. For this image acquisition, sufficient data has been gathered when the majority, for example an amount between 70-90%, of the fill factors are "white" and the remaining portion is "grey". Because a certain percentage of data acquired in only the exhale or inhale phase is acceptable, but it is unpredictable where this will occur in the BICM, it is more efficient to define a stopping criterium for the BICM as a whole rather than for each fill factor individually.

For the example shown in Fig. 4b, the stopping criterium has not been met after the illustrated first scan of the subject. As can be seen from the black area, no data was acquired for a significant number of BICM elements. This compromises the quality of the image that will be reconstructed from the data. To remedy this at least one additional scan will be done before data acquisition could be considered as complete. The additional scan of the subject will be performed with radiation source control instruction that have been updated using the determined fill factors.

The radiation source control instructions can be updated according to several options. For maximum dose saving, the radiation source can be switched on for only those BICM elements where no data has been acquired yet, shown as black in Fig.4a. The radiation source will be switched off for all other BICM elements. This will provide the minimum image data needed to reconstruct an acceptable medial image, but may not provide the optimal image quality. Part of the data is still taken from the shallow breaths of the subject. Alternatively, the radiation source control can be switched on for all BICM elements of the BICM rows that have gaps in data acquisition and switched off for all other rows. In this example, this would be the subject support positions slices 39-68 and 99-113. In this approach the data acquired in these slices with irregular breathing for the first scan can be replaced in full by the same slices with data acquired with regular breathing in the second scan. This will have a somewhat higher dose exposure for the subject, but also result in a better quality reconstructed medical image.

This also illustrates an advantage of the approach according to the current invention. By determining the fill factors for the BICM elements, the image slices that may cause artifacts due to irregular breathing motion can be identified without having to first reconstruct the medical image of the subject. A second scan to acquire additional data to replace the affected slices can be acquired immediately when the first scan is complete.

During the time it takes to perform the imaging scan, the subject may relax a bit more. This can result in a different breathing pattern. Most commonly, patients will inhale and exhale less deeply. Since breathing is still regular, this will usually not compromise image quality. Such a change in breathing pattern can be taken into account in the BICM stopping criterium. This could be taken into account as a possibility prior to start of the image data acquisition, but preferably the stopping criterium can be adjusted during the scan when shallower breathing is detected.

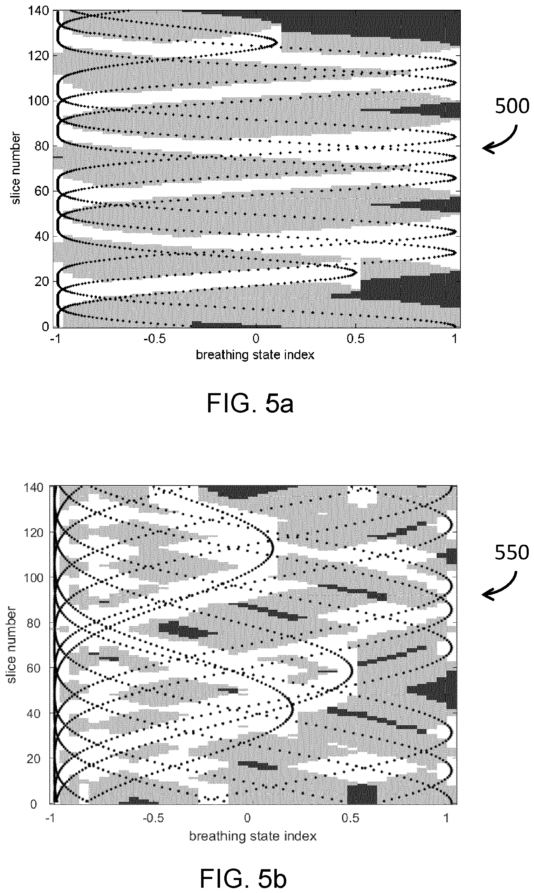

Figs 5a and 5b schematically illustrate examples of a breathing index coverage matrix 500 and 550 for a different image acquisition protocol. For this example, repeated scans in both directions in a high-pitch helical CT scan were used to acquire the CT image. The position of the subject support is indicated as slice number with 140 slices. The breathing state index is provided in normalized units with -1 being the maximum exhale state and 1 being the maximum inhale state. The image acquisition illustrated in Fig. 5a has three passes through the CT scanner and the image acquisition illustrated in Fig. 5b has a higher pitch with ten passes through the scanner within the same time frame.

Like the respiratory signal in Fig. 4b, in Figs. 5a and 5b the subject has some irregular breathing in the form of shallow breath inhalation. The fill factor in these examples again can have three discrete values: no data acquired, shown in black, data acquired either during inhalation or exhalation shown in gray, and data acquired during both inhalation and exhalation shown in white.

As can been seen in these examples, a different type of coverage of the BCIM 500 and 550 can be achieved with this image acquisition protocol. As compared to Fig. 4b, there are more black areas with no data, but they are smaller in size and have a different distribution. Depending on the requirements for the scan and consequently the stopping criterium for the BCIM, this alternative option can result in less additional required scans.

Any of the method steps disclosed herein may be recorded in the form of a computer program comprising instructions which when executed on a processor cause the processor to carry out such method steps. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid-state memory, magnetic tape, a removable computer diskette, a random-access memory "RAM", a read-only memory "ROM", a rigid magnetic disk and an optical disk. Current examples of optical disks include compact disk - read only memory "CD-ROM", compact disk - read/write "CD-R/W", Blu-Ray^{™} and DVD. Examples of a propagation medium are the Internet or other wired or wireless telecommunication systems.

Variations to the disclosed embodiments can be understood and effected by those skilled in the art in practicing the claimed invention, from a study of the drawings, the disclosure, and the appended claims. It is noted that the various embodiments may be combined to achieve further advantageous effects.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A method for acquiring medical image data (200) of a subject, the method comprising the steps of:
- receiving a respiratory signal (130) of the subject and correlating the respiratory signal to a position of a support for the subject to identify one or more breathing index coverage matrix elements (210);
- performing an imaging scan of the subject (220), during which imaging scan the subject is exposed to radiation emitted by a radiation source, which radiation source is controlled by control instructions;
- determining fill factors of the identified one or more breathing index coverage matrix elements (230); and
- updating the radiation source control instructions using the determined fill factors (240).

2. The method according to claim 1, wherein determining the fill factors of the one or more breathing index coverage matrix elements and updating the radiation source control instructions using the determined fill factor take place at least partly simultaneously with performing the imaging scan of the subject (245).

3. The method according to claim 1 or 2, wherein the steps are repeated (255) until the breathing index coverage matrix meets a stopping criterium (250).

4. The method according to claim 3, wherein the stopping criterium is met when each fill factor has reached a coverage threshold.

5. The method according to any of claims 1-4, wherein the radiation source control instructions are instructions for switching the source on or off.

6. The method according to any of claims 1-5, further comprising a step of updating the imaging scan pitch using the determined fill factors.

7. The method according to any of claims 1-6, wherein the fill factor has a number of discrete values.

8. The method according to any of claims 1-6, wherein the fill factor comprises dwell time.

9. The method according to any of claims 1-8, wherein the radiation source is controlled to stop emitting radiation when irregular breathing is detected and to resume emitting radiation when regular breathing resumes.

10. A system for controlling an imaging device to acquire medical image data (120) of a subject, comprising:
- a control unit configured to perform the method according to any of claims 1-9.

11. An imaging arrangement (100) for acquiring medical image data of a subject, comprising:
- an imaging device (110) comprising a controllable source of radiation (108);
- the system according to claim 10 for controlling the imaging device.

12. The imaging arrangement (100) according to claim 11, further comprising a respiratory monitor configured to provide the respiratory signal (130) of the subject.

13. The imaging arrangement according to claim 11 or 12, further comprising an image reconstruction system (140) configured to reconstruct the imaging data to generate an image of the subject disposed in the examination region (106).

14. A computer program product comprising instructions for causing a processor to carry out the method according to any of claims 1-9, when the computer program is executed.
